# EUROPEAN PATENT SPECIFICATION

(11) **EP 1 547 641 B1**
(45) Date of publication and mention of the grant of the patent: **11.03.2015**
(21) Application number: 04030517.9
(22) Date of filing: 22.12.2004
(51) Int. Cl.: A61M 25/10, A61M 16/04

(54) **Balloon control apparatus**
Steuerungsgerät für einen Ballon
dispositif de commande pour un ballonnet

(30) Priority: 25.12.2003 JP 2003430506; 04.11.2004 JP 2004321220
(43) Date of publication of application: 29.06.2005
(73) Proprietor: FUJIFILM Corporation, Tokyo 106-8620 (JP); SRJ Corporation, Kawachi-gun Tochigi (JP)
(72) Inventor: Sekiguchi, Tadashi, Saitama-shi Saitama (JP); Fujikura, Tetsuya, Saitama-shi Saitama (JP); Norinobu, Tomoya, Saitama-shi Saitama (JP)
(74) Representative: Grünecker, Kinkeldey, Stockmair & Schwanhäusser

(56) References cited:
- EP-A- 0 075 153
- US-A- 5 024 668

## Description

### BACKGROUND OF THE INVENTIONS

### Field of the Invention

The present invention relates to a balloon control device, and more particularly to a balloon control device for controlling a balloon installed to an insertion unit of endoscope and an insertion adapter.

### Description of the Related Art

As disclosed in Japanese Patent Application Publication No. 2003-144378, an endoscope system using balloon are developed recently. In this case, the balloon is attached around the tip of an insertion unit of endoscope, or the tip of an insertion adapter in which covers on the insertion unit. Therefore, the insertion unit or the insertion adapter is fixed within the body cavity by expanding the balloon within the body cavity.

The insertion unit and the insertion adapter are provided an air supply intake port communicating with the balloon at the base end of those. The air supply intake port is connected to a balloon control device by a tube. Air is supplied and sucked to the tube at the balloon control device so that the balloon is expanded and contracted.

However, the conventional balloon control device has a possibility that an unclean matter such as body fluid is sucked into the device through the tube when the balloon is broken or when the connection of the tube is bad. In such case, an electromagnetic valve and a pump within the balloon control device are damaged by the unclean matter. In order to cope with this problem, it is necessary that the balloon control device is provided with a fluid reservoir such as a trap. However, whenever the unclean matter is sucked, it is necessary to wash and sterilize the trap. This maintenance work has a problem of taking great deal of time and labor.

US 5 024 668 A relates to a retrograde perfusion system having a catheter and a driver to inject retroperfusate into a coronary sinus.

### SUMMARY OF THE INVENTION

The present invention was contrived in view of such circumstances, and an object thereof is to provide a balloon control device capable of preventing unclean matter from being sucked into it and permitting easy maintenance in terms of washing and sterilization.

To achieve the above-stated object, a first aspect of the present invention is directed to a balloon control device according to claim 1.

According to the first aspect of the invention, since the gas/liquid separator is provided with the fluid channel of the balloon control device, the sucked liquid is separated from air by the gas/liquid separator. Therefore, it is possible to prevent the liquid from being sucked into the balloon control device.

A second aspect of the invention is directed the balloon control device as defined in the first aspect, characterized in that the gas/liquid separator is one of a gas/liquid separation filter, a water-absorbent polymer member, a paper fiber member, a water-absorbent fiber member, and a liquid reservoir tank.

A fourth aspect of the invention is directed the balloon control device as defined in the third aspect, characterized in that the tube is linked to the gas/liquid separator with a lure lock mechanism. Therefore, according to the invention, the gas/liquid separator and the tube can be easily linked with each other in an airtight state.

A fifth aspect of the invention is directed the balloon control device as defined in the fourth aspect, characterized by further comprising a hollow portion for installing the gas/liquid separator into the body. Therefore, according to the sixth aspect of the invention, since the gas/liquid separator is installed in the hollow portion in the body of the balloon control device, the gas/liquid separator does not protrude when it is installed to the body, and the device can be made compact.

A sixth aspect of the invention is directed the balloon control device as defined in the first and third aspects, characterized in that the gas/liquid separator is formed by laminating a water-absorbent member on inner surface of the tube. Therefore, according to the sixth aspect of the invention, as it is required only to provide the tube on the channel by which the balloon and the balloon control device communicate with each other, the device can be simplified and made compact.

Eighth and tenth aspects of the invention are directed the balloon control device as defined in the first, third, and sixth aspects, characterized by further comprising a liquid detector for detecting liquid in the gas/liquid separator. Therefore, according to the Eighth to eleventh aspects of the invention, since the arrival of any liquid at the liquid detecting device can be known, the timing for the replacement, maintenance or washing of the gas/liquid separator can be known accurately.

Eleventh and thirteenth aspects of the invention is directed the balloon control device as defined in the eighth to tenth aspects, characterized in that the liquid detector has a component to change color when liquid is detected. Therefore, according to the eighth aspect of the invention, the presence or absence of liquid can be checked by a color change of the liquid detecting device.

Therefore, according to the balloon control device of the present invention, it is possible to prevent liquid in fluid from getting into the balloon control device by disposing the gas/liquid separator, effectively.

### BRIEF DESCRIPTION OF THE DRAWINGS

The nature of this invention, as well as other objects and advantages thereof, will be explained in the following with reference to the accompanying drawings, in which like reference characters designate the same or similar parts throughout the figures and characterized in that:
Fig. 1 is a schematic perspective diagram of an endoscope system according to an embodiment of the present invention;
Fig. 2 is a perspective diagram showing a tip portion of an insertion unit according to an embodiment of the present invention;
Fig. 3 is a schematic block diagram of an endoscope system according to an embodiment of the present invention;
Fig. 4 is a perspective view of a balloon control device according to an embodiment of the present invention;
Fig. 5 is a side cross-sectional view of a filter unit according to an embodiment of the present invention;
Fig. 6 is a schematic perspective view of the endoscope system with a balloon installed to an insertion adapter;
Fig. 7 is a schematic perspective view of the endoscope system with a balloon installed to an insertion adapter and an insertion unit;
Fig. 8 is a schematic plan view of a gas/liquid separator according to another embodiment of the present invention;
Fig. 9 is a schematic plan view of a gas/liquid separator according to still another embodiment of the present invention;
Fig. 10 is a schematic plan view of a gas/liquid separator according to further embodiment of the present invention;
Fig. 11 is a cross-sectional view of a gas/liquid separator according to still further embodiment of the present invention;
Fig. 12 is a schematic plan view of a gas/liquid separator equipped with a liquid detecting device according to an embodiment of the present invention; and
Fig. 13 is a schematic plan view of a gas/liquid separator equipped with a liquid detecting device according to another embodiment of the present invention.

### DETAILED DESCRIPTION OF THE PREFERRED EMBODIMENTS

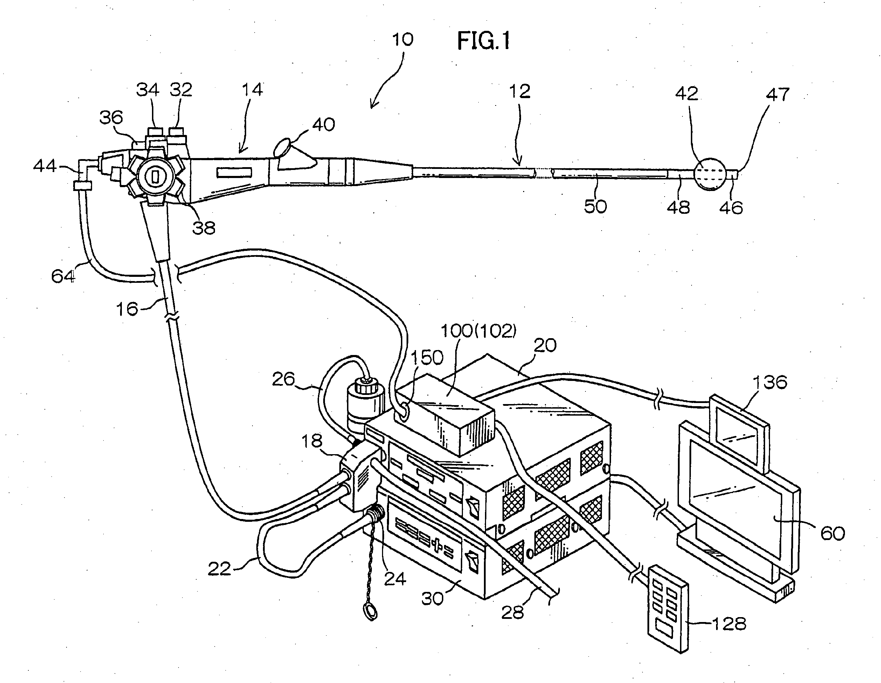

Fig. 1 is a schematic perspective view of an endoscope system according to an embodiment of the present invention. As shown in Fig. 1, the endoscope system is mainly comprised an endoscope 10, a light equipment 20, a processor 30, and a balloon control device 100.

The endoscope 10 is composed of an insertion unit 12 to be inserted into a body cavity, and a handy control unit 14 linked to the insertion unit 12. The insertion unit 12 and the handy control unit 14 are connected by a universal cable 16. While an LG connector 18 is provided at the tip of the universal cable 16, the LG connector 18 is connected to the light equipment 20. While an electrical connector 24 is connected to the LG connector 18 via a cable 22, the electrical connector 24 is linked to the processor 30. A gas feed/water feed tube 26 for supplying air or water, and a suction tube 28 for sucking air and body fluid are connected to the LG connector 18.

The handy control unit 14 is equipped with a gas feed/water feed button 32, a suction button 34 and a shutter release button 36, and is also provided with a pair of angle knobs 38 and 38, and a forceps insertion unit 40. At the base end portion of the handy control unit 14, a feed/suction port 44 is provided to facilitate gas supply to and gas suction from a balloon 42 as described later. The following description supposes the use of air as gas, but some other gas, such as inert gas, may be used as well.

The insertion unit 12 is composed of a tip portion 46, a curved portion 48, and a soft portion 50. The curve of the curved portion 48 is controlled remotely by turning the pair of angle knobs 38 and 38 in the handy control unit 14. Therefore, it is possible to turn the tip face 47 of the tip portion 46 into a desired direction.

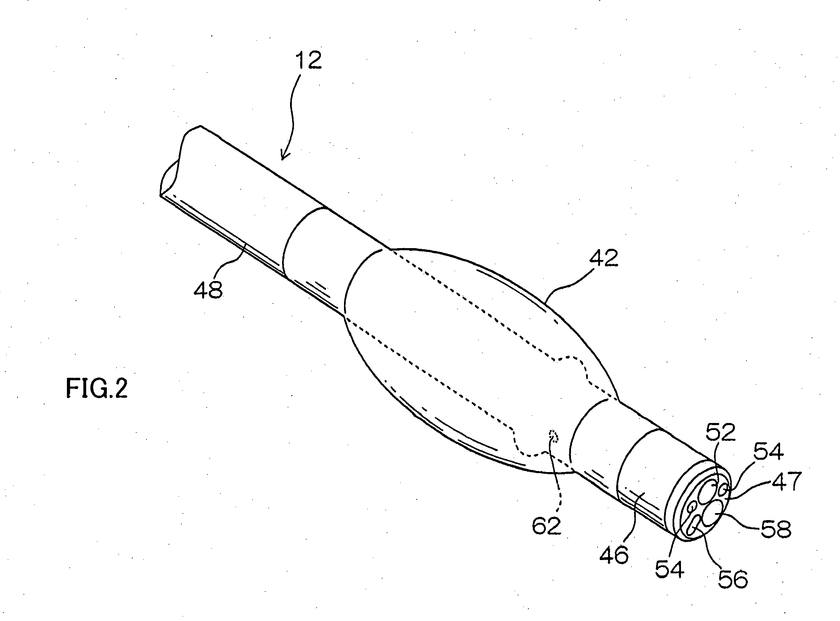

As shown in Fig. 2, the tip face 47 of the tip portion 46 is provided with an observational optical unit 52, a plurality of illuminating optical units 54, a gas feed/water feed nozzle 56, and a forceps port 58. As not shown in Fig. 2, a CCD is arranged behind the observational optical unit 52 (i.e., opposite to the side of the tip face 47 in the observational optical unit 52), and a signal cable is connected to a substrate holding the CCD. The signal cable is extended to the electrical connector 24 while inserted into the insertion unit 12, the handy control unit 14 and the universal cable 16 shown in Fig. 1, and is connected to the processor 30. In this case, an observing image taken at the observational optical unit 52 is converted into an electrical signal by forming an image on the light receiving face of the CCD, and then the electrical signal is supplied to the processor 30 via the signal cable to be converted into an image signal. Therefore, the observed image is displayed on a monitor 60 connected to the processor 30.

The emitting end of a light guide (not shown) is arranged behind the illuminating optical units 54 in Fig. 2 (i.e., opposite to the side of the tip face 47on the illuminating optical units 54). The light guide is inserted into the insertion unit 12, the handy control unit 14, and the universal cable 16 in Fig. 1. The incidence end is arranged on the LG connector 18. Therefore, the illuminating optical units 54 irradiate the illuminating light emitted from the light equipment 20 via a light guide (not shown).

The gas feed/water feed nozzle 56 (referred in Fig. 2) is connected to the gas feed/water feed tube 26 while the gas feed/water feed nozzle 56 (referred in Fig. 2) is connected to a valve (not shown) manipulated with the gas feed/water feed button 32. Therefore, air or water is discharged from the gas feed/water feed nozzle 56 toward the observational optical unit 52 by operating the gas feed/water feed button 32.

While the forceps port 58 in Fig. 2 is linked to a forceps insertion unit 40, the forceps port 58 is connected to a valve (not shown) operated by the suction button 34. In addition, the forceps port 58 is further connected to the suction tube 26. Therefore, a lesion and the like is sucked from the forceps port 58 by operating the suction button 34, and a treating tool (not shown) is guided out through the forceps port 58 by inserting through the forceps insertion unit 40.

As shown in Fig. 2, the balloon 42 consisting of an elastic material such as rubber is installed around the part of the insertion unit 12. It is formed in a substantially cylindrical shape with its two ends narrowed. The balloon 42 is fixed by fixing the two ends to the insertion unit 12 after it is inserted through the insertion unit 12. The two ends of the balloon 42 is fixed by, for instance, winding a thread around each end part of the balloon 42, and both ends of the balloon 42 are thereby fastened to the full outer circumference of the insertion unit 12. Instead of winding a thread, a fixed ring of rubber, and the like may be installed onto each end of the balloon 42.

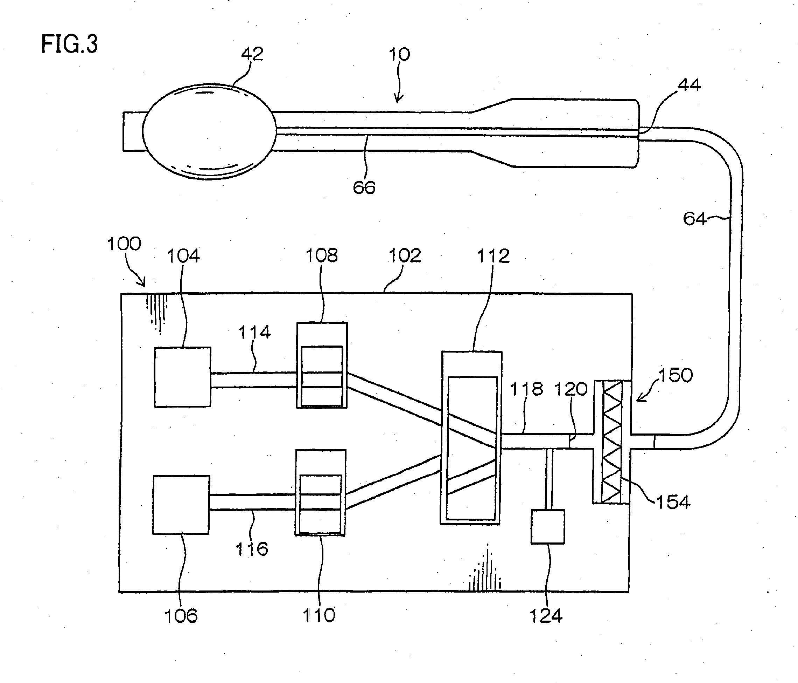

A ventilation port 62 is formed in the installing position for the balloon 42 on the insertion unit 12. This ventilation port 62 is connected to the feed/suction port 44 via a tube 66 (see Fig. 3) inserted into the insertion unit 12. The tip of a tube 64 is linked to the feed/suction port 44, and the base end of this tube 64 is connected to the balloon control device 100 to be described in further detail afterwards. The balloon control device 100 feeds air to and sucks air from the balloon 42 via the tubes 64 and 66, and thereby controls the expansion or contraction of the balloon 42. The balloon 42 inflates into a substantially spherical shape when fed with air and sticks to the outer surface of the insertion unit 12 when air is sucked out of it.

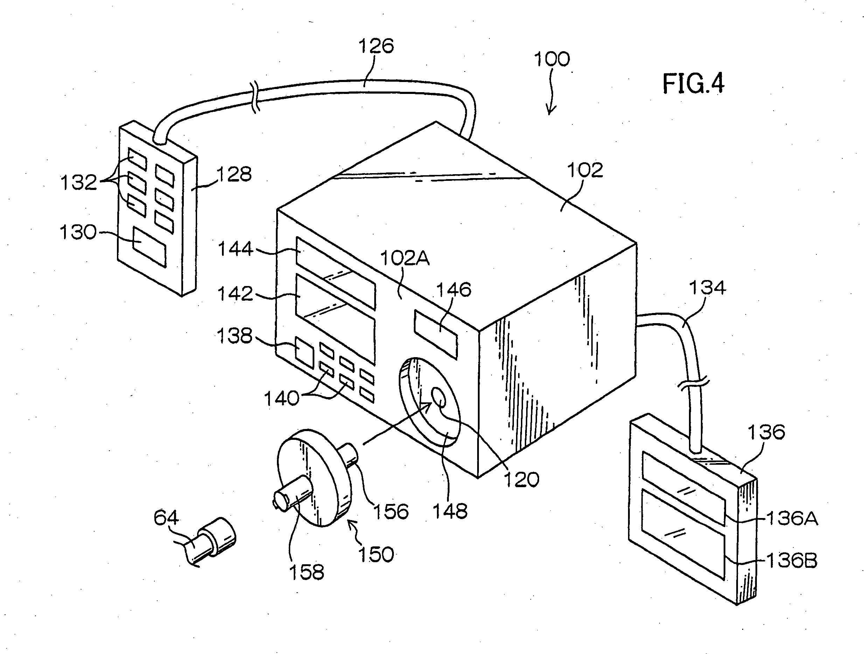

Fig. 3 is a schematic block diagram of an endoscope system according to an embodiment of the present invention, showing air channel simply. Fig. 4 is a perspective view of a balloon control device 100 according to an embodiment of the present invention.

As shown in Fig. 3, a gas feed pump 104, a suction pump 106, a first on/off electromagnetic valve 108, a second on/off electromagnetic valve 110, and a change-over electromagnetic valve 112 are disposed within the body 102 of the balloon control device 100. While a gas feed duct 114 is connected to the gas feed pump 104, the gas feed duct 114 is connected to the change-over electromagnetic valve 112 via the on/off electromagnetic valve 108. While a suction duct 116 is connected to the suction pump 106, the suction duct 116 is connected to the change-over electromagnetic valve 112 via the second on/off electromagnetic valve 110. While the change-over electromagnetic valve 112 is connected to a connection port 120 via a duct 118, the connection port 120 is connected to the tube 64 via a filter unit 150 as described later.

The change-over electromagnetic valve 112 has a configuration in which the duct 118 is connected to one of the gas feed duct 114 and the suction duct 116. For example, in the case feeding air to the balloon 42, the change-over electromagnetic valve 112 is controlled to connect the gas feed duct 114 with the duct 118. On the other hand, in the case sucking air from the balloon 42, the change-over electromagnetic valve 112 is controlled to connect the suction duct 116 with the duct 118. Furthermore, when electric power supply is off, the change-over electromagnetic valve 112 makes the suction duct 116 connected with the duct 118, so as to prevent the balloon 42 from expanding in an unusual situation such as a power failure.

The first and second on/off electromagnetic valves 108 and 110 control the opening and closing of the gas feed duct 114 and the suction duct 116, respectively. When the power supply is off, the gas feed duct 114 and the suction duct 116 are closed. It is possible that feeding air and operating to suction are temporarily suspended by controlling on/off of the first and second on/off electromagnetic valves 108 and 110. When the value of a pressure gauge 124 reached the set level in expanding the balloon 42, air supply is temporarily suspended by closing the first on/off electromagnetic valve 108. On the other hand, when the value of the pressure gauge 124 falls below the set level, air supply is resumed by opening the first on/off electromagnetic valve 108. Therefore, the internal pressure of the balloon 42 can be securely maintained at the set level. In the case in which the diameter of the tube 66 is smaller than that of the tube 64, the value of the pressure gauge 124 is different to the actual internal pressure of the balloon 42. Therefore, the first on/off electromagnetic valve 108 as described above may maintain the internal pressure of the balloon 42 at the setting value, exactly. In the same way, when the balloon 42 is to be contracted, the suction of air is temporarily stopped and resumed by closing or opening the second on/off electromagnetic valve 110, so as to maintain the internal pressure of the balloon 42 at the setting value.

As shown in Fig. 4, a remote controller 128 is connected to the body 102 of the balloon control device 100 via a cable 126. The remote controller 128 is provided with a power switch 130 and a plurality of first control buttons 132 for altering the setting value of the pressure in the balloon 42, and for turning the supplying and sucking of air.

A balloon monitor 136 is connected to the body 102 via a cable 134. The balloon monitor 136 is provided with a status display unit 136A and an error display unit 136B. The status display unit 136A displays the status of the expanded or contracted balloon 42, and the error display unit 136B displays an error message in the error. Since the balloon monitor 136 is detachably installed to the monitor 60 in Fig. 1, it is possible to check the status of balloon 42 and any error message on the balloon monitor 136 while the image taken by the endoscope 10 is observed on the monitor 60. The balloon control device 100 according to the invention is not limited to above, and the status of balloon 42 and the error message may be displayed on the monitor 60 instead of the balloon monitor 136. The remote controller 128 may be provided with a display screen for displaying the status of balloon 42 and the error message. Furthermore, the status of balloon 42 and any error message may be displayed on the monitor 60 as the superimpose on the endoscopic image by entering image signal from the endoscope 10 into the balloon control device 100.

As shown in Fig. 4, a power switch 138 and various control buttons 140 are disposed on the front panel 102A of the body 102. Since the second control buttons 140 have the same functions as the first control buttons 132 of the remote controller 128, it is possible that the balloon control device 100 is operated by the first and second control buttons 132 and 140.

In addition, the front panel 102A is provided with a balloon display unit 142 for displaying the status of balloon 42 and an error display unit 144 for displaying any error message. Therefore, it is possible to check the status of balloon 42 and the error message by looking at the front panel 102A. The front panel 102A is also provided with a pressure display unit 146 to enable the value of the pressure gauge 124 (referred in Fig. 3) to be displayed.

A disk-shaped concave receptacle 148 is formed in the front panel 102A in the position of connection with the tube 64, and a filter unit 150 is detachably installed and accommodated in the receptacle 148.

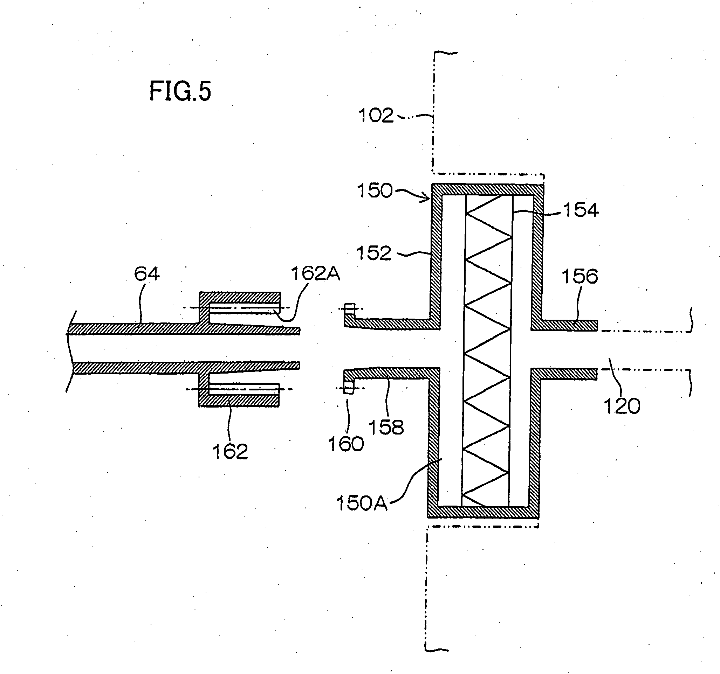

As shown in Fig. 4 and 5, the filter unit 150 is composed of a hollow casing 152 and a disk-shaped membrane filter 154 (hereinafter referred to as filter 154 simply). While the casing 152 has substantially a disk shape, the filter 154 is arranged within the casing 152. A cylindrical linking portion 156 is projected on the back central part of the casing 152 (i.e., the side toward the body 102). When the linking portion 156 is fitted into the connection port 120 of the body 102, the interior of the casing 152 is connected to the connection port 120 while the casing 152 installed to the body 102 is accommodated in the receptacle 148.

The method of fixing the casing 152 to the body 102 is not limited to above embodiment. For example, it may be suitable that a catching projection formed on the outer circumference of the casing 152 is set to a catching groove formed on a side of the receptacle 148. In the case of the connection port 120 consisting of a rubber or some other elastic member, it is possible to fix the connection port 120 in the casing 152 stability by the elasticity of those when inserting the connection port 120 into the linking portion 156. Furthermore, the connection port 120 and the linking portion 156 may be linked with a lure lock mechanism as described later.

A cylindrical linking portion 158 is formed by projecting on the central part of the surface of the casing 152. It is preferable that the linking portion 158 has a structure to ensure linking with the tube 64 in an airtight state, and for example, a lure lock mechanism is used in the embodiment. In other words, as shown in Fig. 5, a flange 160 is protrusively formed on the outer circumference of the end part of the linking portion 158, and a female screw 162A with which the flange 160 is to engage is formed in the inner circumferential face of the connector 162 of the tube 64. By engaging the flange 160 with the female screw 162A, the linking portion 158 and the connector 162 are linked in a sustained airtight state. Incidentally, a female screw may as well be formed on the linking portion 158, and a flange formed on the connector 162 of the tube 64.

A filter 154 arranged within the casing 152 is formed at an outer diameter equal to the inner diameter of the casing 152, and the whole fluid passing the inside of the casing 152 passes the filter 154. The filter 154 is so configured as not to pass liquid but to pass only gas. As any liquid passing the casing 152 is collected by the filter 154, liquid can be prevented from passing the casing 152. A radial straightening device can also be disposed within the casing 152 so that air can pass uniformly over the whole face of the filter 154.

Next, the action of the balloon control device 100 as configured above is described following.

The balloon control device 100 can control the expansion, the contraction, and the stopping according to the balloon 42, by manipulating the first control buttons 132 and the second control buttons 140.

For example, in the case of expanding the balloon 42, the first on/off electromagnetic valve 108 is opened while the gas feed pump 104 in Fig. 3 is driven, and then the change-over electromagnetic valve 112 is changed over to the side of the gas feed duct 114. Therefore, air fed from the connection port 120 is supplied to the balloon 42 via the tubes 64 and 66.

Next, the pressure value in the balloon 42 is controlled at the set level by opening and closing the first on/off electromagnetic valve 108 according to the value of the pressure gauge 124. Therefore, the balloon 42 is expanded at the internal pressure controlled to the set level.

In the case of contracting the balloon 42, the second on/off electromagnetic valve 110 is opened while the suction pump 106 is driven, and then the change-over electromagnetic valve 112 is changed over to the side of the suction duct 116. Therefore, air in the balloon 42 is sucked into the connection port 120 via the tubes 64 and 66.

Next, the internal pressure of the balloon 42 is controlled at the set level by opening and closing the second on/off electromagnetic valve 110 according to the value of the pressure gauge 124. Therefore, the balloon 42 is contracted at the internal pressure controlled to the set level.

In the case of unusual situation during the expanding and contracting operation as described above, while the balloon control device 100 is stopped the operation, an error message is displayed during the unusual situation on the error display units 144 and 136B. In this case, it is preferable for the type of the error that has occurred to be identified and to display the identified error type. For example, when the value of the pressure gauge 124 is above the preset threshold of unusual pressure, the situation is identified as one of an unusual pressure resulting from the working of an undue force on the balloon 42, and that finding is displayed on the error display units. On the other hand, when the value of the pressure gauge 124 fails to vary to the set level in a prescribed period of time, the cause is determined to be faulty connection of the tube 64, and that finding is displayed on the error display units.

Furthermore, when the first and/or second on/off electromagnetic valves 108 and/or 110 are frequently changed over in a prescribed period of time (e.g. 40 seconds), it will be judged that the balloon 42 has been broken and an error message to that effect will be displayed on the error display units. In the case in which the balloon 42 is broken, the internal pressure of the balloon 42 will soon vary even if the value of the pressure gauge 124 reaches the set value and the first and second on/off electromagnetic valves 108 and 110 are closed. Therefore, it is necessary to open the first and second on/off electromagnetic valves 108 and 110 again for feeding and sucking air. In this time, the first and second on/off electromagnetic valves 108 and 110 repeated the opening and closing. Therefore, it is possible to detect the breaking of the balloon 42 by counting how many times they are opened and closed.

In the case in which the balloon 42 is broken during the contracting process, there is some possibility of sucking liquid such as body fluid into the balloon 42 via the tubes 64 and 66. Therefore, the conventional balloon control device has a problem in which the change-over electromagnetic valve 112 and the suction pump 106 in the body 102 are damaged by the sucked liquid. In the embodiment of present invention, it is possible to solve such a problem by providing with the filter unit 150. In other words, the liquid sucked via the tube 64 is removed by the filter 154 when it has reached the filter unit 150. The removed liquid is stored in a space 150A (referred in Fig.5) located closer to the linking portion 158 than the filter 154 in the casing 152. Therefore, there is no fear of liquid being sucked into the body 102, and the change-over electromagnetic valve 112 and the suction pump 106 in the body 102 are prevented from damage.

As described above, the balloon control device 100 according to embodiment of the invention can prevent liquid from being sucked into the body 102 by virtue of the presence of the filter unit 150 between the body 102 and the tube 64.

In addition, since the filter unit 150 is disposed to be detachable from the body 102 in the embodiment of the invention, the filter unit 150 can be removed from the body 102 to facilitate its sterilization and disinfection. Therefore, the filter unit 150 can be replaced as a disposable item.

In the embodiment, since the filter unit 150 is linked to the tube 64 with a lure lock mechanism, it is possible to install the filter unit 150 easy by pressing the base end of the tube 64 into the linking portion 158 of the filter unit 150 while rotating it, so that air tightness can be achieved in this process. In addition, the tube 64 can be easily taken off the filter unit 150 by pulling the base end of the tube 64 while rotating it. Therefore, the tube 64 can be removed from the filter unit 150 to facilitate sterilization and disinfection of the tube 64 alone.

Additionally, although the filter unit 150 and the tube 64 are linked with each other by using a lure lock mechanism in the embodiment described above, they may be linked in some other way. For example, since the filter unit 150 is alternately formed circular convexes and concaves on the outer circumferential face of the linking portion 158, it is possible to snapped the tube 64 on outside the linking portion 158.

Furthermore, though the balloon control device 100 described above is supposed to control a balloon 42 installed to the insertion unit 12 of the endoscope 10, it can also be applied as a device for controlling a balloon installed to an insertion adapter.

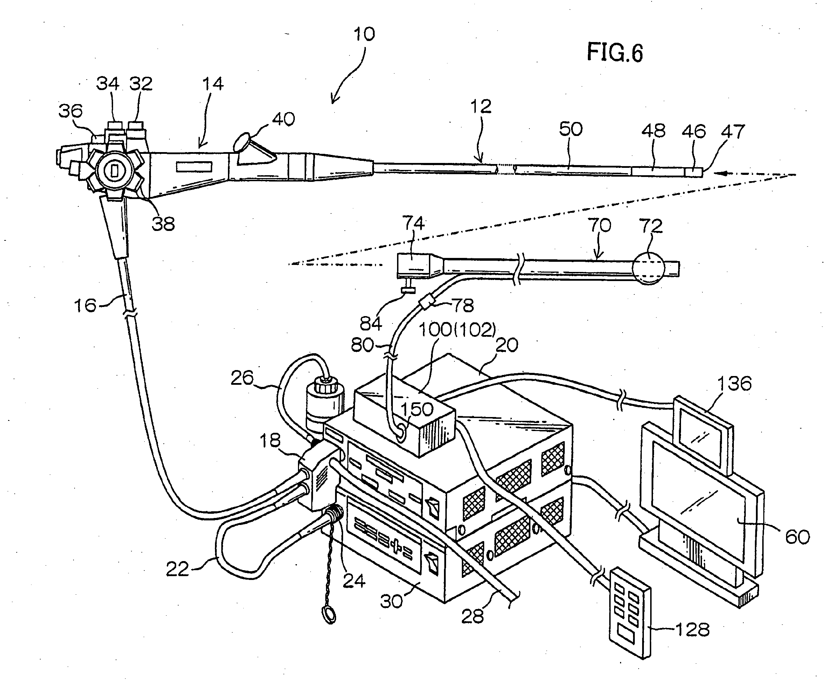

Fig. 6 is a schematic perspective view of the endoscope system with a balloon installed to an insertion adapter.

As shown in Fig. 6, an insertion adapter 70 is formed by protecting the inside and outside of a resin tube, consisting of urethane or the like, with lubricant coats, and exerts a righting moment when an external force is applied to it from the outer circumferential face. The inner diameter of the insertion adapter 70 is greater than the outer diameter of the insertion unit 12 of the endoscope 10, so that the insertion unit 12 can be inserted into the insertion adapter 70.

A ring (not shown), consisting of an X-ray intercepting member of metal or some other material, is disposed at the tip of the insertion adapter 70, so that the tip position of the insertion adapter 70 can be recognized in roentgenoscopy.

A second balloon 72 made of rubber is installed around the tip of the insertion adapter 70. The second balloon 72 is cylindrically shaped like the first balloon 42 shown in Fig. 3, and both ends 72A of the second balloon 72 are fixed to the insertion adapter 70. Reference numeral 84 in Fig. 6 denotes a feed port for feeding a lubricant, such as water. By feeding a lubricant through the feed port 84, the friction between the insertion adapter 70 and the insertion unit 12 can be reduced.

A tube 76 stuck to the outer surface of the insertion adapter 70 connected to the second balloon 72, and the tip portion of a tube 80 is detachably linked to a connector 78 disposed at an end of this tube 76. The base end of the tube 80 is linked to the balloon control device 100, which feeds air to or sucks air from the tube 80 and can control the air pressure in that process. Therefore, it is possible to feed air to or suck air from the balloon 72.

In the balloon control device 100, as described above, the filter unit 150 is detachably installed to the front panel 102A of the body 102. The base end of the tube 80 is linked to the linking portion 158 of the filter unit 150. The linking portion 158 and the tube 80 are linked to each other by using a lure lock mechanism for instance.

In the endoscope system configured as described above, too, as the filter unit 150 is disposed on the linking portion between the balloon control device 100 and the tube 80, the filter unit 150 can remove air sucked by the tube 80, and thereby prevent the liquid from being further sucked into the body 102. Therefore, the balloon control device 100 can also be applied as a device for controlling a balloon 72 installed to an insertion adapter 70.

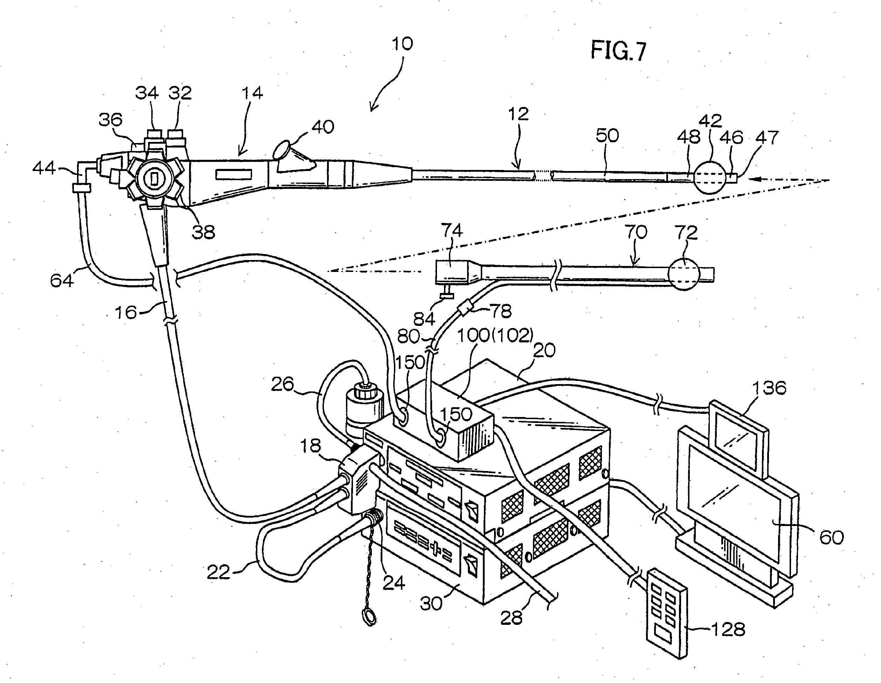

Fig. 7 is a schematic perspective view of the endoscope system with a balloon installed to an insertion adapter and an insertion unit. As shown in Fig. 7, in the endoscope system, while a first balloon 42 is installed to the insertion unit 12, a second balloon 72 is installed to the insertion adapter 70. The balloon control device 100 and two filter units 150 and 150 are detachably installed to the body 102. While the tube 64 connected to the balloon 42 is linked to the first filter unit 150, the tube 80 connected to the second balloon 72 is linked to the second filter unit 160. Within the body 102, two lines of air pressure control devices are provided to enable the tube 64 and the tube 80 to feed and suck air, respectively.

In such above endoscope system, liquid sucked via the tubes 64 and 80 can be also removed by the filter units 150 and 150 to prevent liquid from being further sucked into the body 102. Furthermore, since the filter units 150 and 150 are detachably installed, maintenance work on the filter units 150, including washing and sterilization, can be easily accomplished.

Although the embodiment of the invention described above uses the filter units 150 as a gas/liquid separator, the usable gas/liquid separator are not limited to those. For example, water-absorbent polymer, a porous material consisting of polytetrafluoroethylene or the like, paper fibers such as Japanese rice paper, water-absorbent fibers such as cotton wool can be used in place of the filter 154.

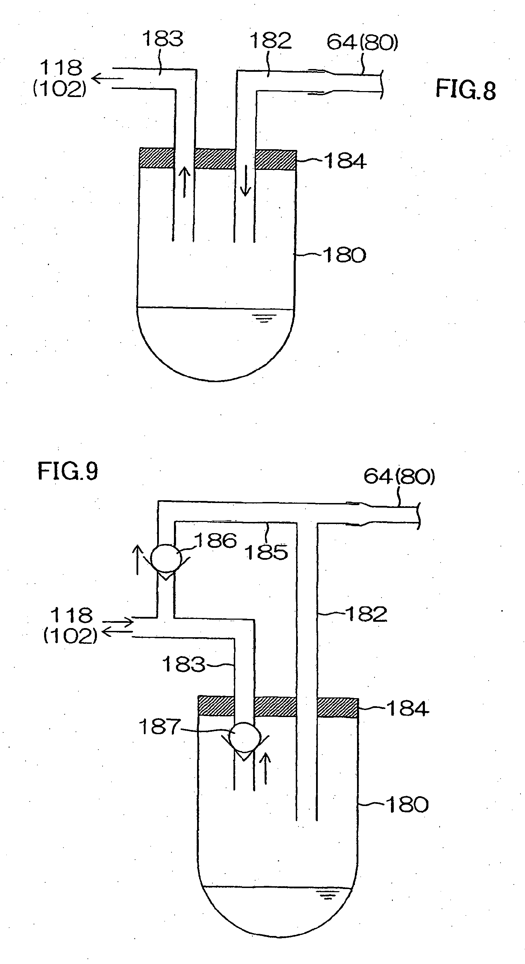

As shown in Fig. 8, a liquid reservoir tank 180 to serve as the trap for liquid can also be used as a gas/liquid separator. The liquid reservoir tank 180 is so installed that two pipes 182 and 183 penetrate a lid 184. The lower ends of the pipes 182 and 183 are arranged above the liquid surface. The tube 64 (or the tube 80) is connected to one pipe 182, and the other pipe 183 communicates with the duct 118 (see Fig. 3). When liquid and gas flow from the tube 64 into the liquid reservoir tank 180 configured in this way via the pipe 182, liquid is left in the reservoir tank 180, and only gas is extracted from the other pipe 183. Therefore, liquid can be prevented from entering into the body 102.

The reservoir tank 180 may as well be configured as shown in Fig. 9. The pipes 182 and 183 for the reservoir tank 180 in Fig. 9 are connected to a bypass pipe 185, and the bypass pipe 185 is provided with a non-return valve 186 for preventing flow from the pipe 182 to the pipe 183. The pipe 183 in the reservoir tank 180 is provided with a non-return valve 187 for preventing flow to the reservoir tank 180. When fluid from the first or second tube 64 or 80 is sucked into the reservoir tank 180 configured in this way, the fluid flows into the reservoir tank 180 via the pipe 182 without passing the bypass pipe 185, and liquid is separated in the tank and only gas is sucked from the pipe 182. Therefore, it is possible to prevent liquid from flowing into the body 102. When air is supplied from the body device 102 to the pipe 183, the air does not flow into the reservoir tank 180 but into the first or second tube 64 or 80 via the bypass pipe 185. Accordingly, since the air flows detouring the reservoir tank 180, it is not pressurized in the reservoir tank 180, but liquid (body fluid or the like) depositing in the reservoir tank 180 can be prevented from flowing back toward the endoscope 10.

In the above-described embodiment, the gas/liquid separator (the filter units 150) are disposed in the connecting parts between the body 102 and the tubes 64 and 80, but the positions of the gas/liquid separator are not limited to those. It is possible to arrange the gas/liquid separator on any appropriate positions in the channel of air sucked from the first and second balloons 42 and 72. Therefore, they can be arranged midway on the duct 118 or the suction duct 116 within the body 102 or midway on the first and second tubes 64 and 80.

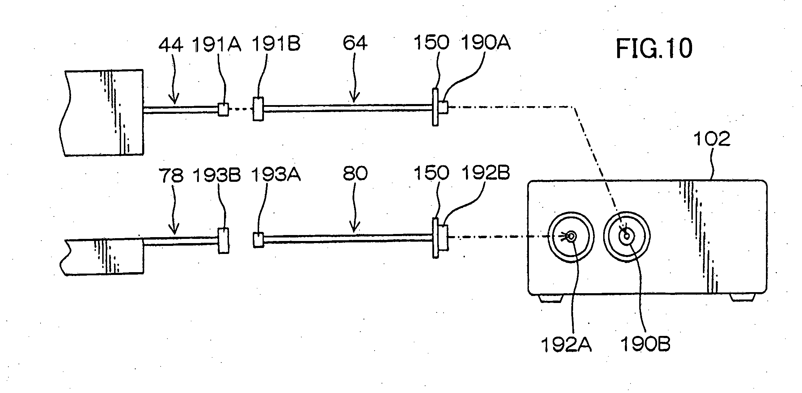

As shown in Fig. 10, it is also preferable that the filter units 150 are arranged integrally at ends of the tubes 64 and 80. It is possible to reduce the frequency of linking of the connectors by integral arrangement. In this case, it is preferable to provide the tubes 64 and 80 shown in Fig. 10 with structures against wrong piping to prevent erroneous linking. Therefore, the tube 64 is provided with a lure taper-shaped male side coupling 190A at the end toward the filter unit 150 and with a female side coupling 191B at the other end. The tube 80 is provided with a female side coupling 192B on the filter 150 side and with a male side coupling 193A at the other end. The bodies 102 with which the couplings 190A and 192B are to be linked are provided with matching female side couplings 190B and 192A. The feed/suction port 44 and the connector 78 with which the couplings 191B and 193A are to be linked are provided with matching male side coupling 191A and female side coupling 193B. Therefore, the configuration in which the male and female couplings are reversely arranged between the tubes 68 and 80 can serve to prevent wrong linking.

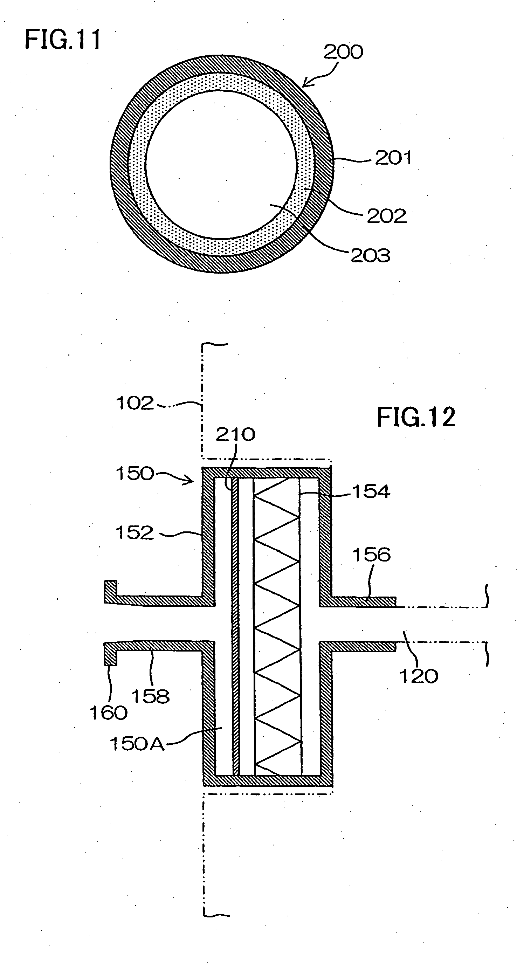

Furthermore, it is preferable to dispose the gas/liquid separator in tubes constituting air channels. In other words, it is preferable to provide the gas/liquid separator within the tubes constituting the duct 118 and the suction duct 116 or the tubes 64 and 80. In this case, it is preferable to use double-structured tubes 200 each consisting of a waterproof tube shell 201 and a water-absorbent member 202 inside it as shown in Fig. 11. Therefore, by the above configuration, it is possible to secure an air channel in the central part 203 of each tube 200 and to separate liquid flowing through the tube 200 by having it absorbed by the water-absorbent member 202.

More over, it is preferable for the gas/liquid separator to be provided with a liquid detection device for detecting liquid. For example, the filter unit 150 shown in Fig. 12 comprises a liquid detecting paper 210 in the space 150A located closer to the linking portion 158 than the filter 154. The liquid detection paper 210 has a property to change color when wetted with liquid. For example, as the liquid detection paper 210, it is preferable to accept the cobalt chloride water whose color changes from blue to red when wetted with water, and a water finding test paper whose color is changed by water from white to blue. A casing 152 of the filter unit 150 is made of a transparent or translucent material so that the liquid detection paper can be recognized from outside. The transparent or translucent portion may constitute the entire casing 152, or a portion thereof through which the liquid detection paper 210 can be viewed. The filter unit 150 configured as described above can detect the entrance of liquid into the casing 152 because the liquid detecting paper 210 changes color as a reaction to the entrance of liquid into the casing 152. When liquid is detected, the paper is subjected to maintenance such as replacement or washing after use. It is also conceivable to use a filter 154 for gas/liquid separation changed color thereof when wetted with liquid.

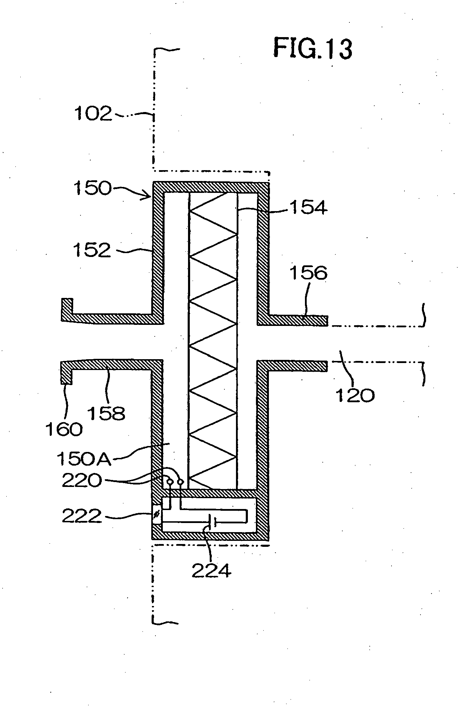

Furthermore, the liquid detection device is not limited to what is described above, but may be configured as shown in Fig. 13. The filter unit 150 shown in Fig. 13 has two terminals 220 and 220 arranged close to each other in the space 150A. These two terminals 220 and 220 are electrically connected to an alarm lamp 222 and a power source 224. When water drips come into contact with the two terminals 220 and 220, an electric current flows to light the alarm lamp 222. Therefore, a liquid detection device configured above can also detect the presence of liquid.

It should be understood, however, that there is no intention to limit the invention to the specific forms disclosed, but on the contrary, the invention is to cover all modifications, alternate constructions and equivalents falling within the scope of the invention as expressed in the appended claims.

## Claims

1. A balloon control device (100) configured to control an expansion and contraction of a balloon (42, 72) fixed to one of an insertion unit (12) of endoscope (10) and an insertion adapter (70) by supplying and suctioning of gas to the balloon (42, 72), **characterized by** comprising:
a main body (102) of the balloon control device (100);
a tube (64, 80) connecting the balloon control device (100) to the balloon (42, 72);
a coupling (191, 193) for connecting the tube (64, 80) and the main body (102); and
a gas/liquid separator (150, 180, 200) which is set to the coupling and is configured to make gas pass without passing the fluid in order that the fluid flowed in from the side of the balloon is prevented from flowing to the side of the balloon control device.

2. The balloon control device (100) as defined in claim 1, wherein the gas/liquid separator (150, 180, 200) is one of a gas/liquid separation filter, a water-absorbent polymer member, a paper fiber member, a water-absorbent fiber member, and a liquid reservoir tank.

3. The balloon control device (100) as defined in claim 1, wherein the tube (64, 80) is linked to the gas/liquid separator (150) with a lure lock mechanism.

4. The balloon control device (100) as defined in claim 3, further comprising a hollow portion configured to install the gas/liquid separator (150) into the body (102).

5. The balloon control device as defined in claim 1, wherein the gas/liquid separator (200) is formed by laminating a water-absorbent member on inner surface of the tube (64, 80).

6. The balloon control device (100) as defined in claim 1, further comprising a liquid detector (210, 220) configured to detect liquid in the gas/liquid separator (150).

7. The balloon control device (100) as defined in claim 5, further comprising a liquid detector (210, 220) for detecting liquid in the gas/liquid separator (150).

8. The balloon control device (100) as defined in claim 6, wherein the liquid detector (210, 220) has a component to change color when liquid is detected.

9. The balloon control device (100) as defined in claim 7, **characterized in that** the liquid detector (210, 220) has a component to change color when liquid is detected.

## Patentansprüche

1. Ballon-Steuervorrichtung (100), die so eingerichtet ist, dass sie Vergrößerung und Verkleinerung eines Ballons (42, 72), der an einer Einführeinheit (12) eines Endoskops (10) oder einem Einführadapter (70) befestigt ist, durch Zuführen und Absaugen von Gas zu/aus dem Ballon (42, 72) steuert, **dadurch gekennzeichnet, dass** sie umfasst:
einen Hauptkörper (102) der Ballon-Steuervorrichtung (100);
eine Röhre (64, 80), die die Ballon-Steuervorrichtung (100) mit dem Ballon (42, 72) verbindet;
eine Kupplung (191, 193), die die Röhre (64, 80) mit dem Hauptkörper (102) verbindet; und
eine Gas-/Flüssigkeits-Trenneinrichtung (150, 18, 200), die an der Kupplung angeordnet und so eingerichtet ist, dass sie Gas durchlässt, ohne das Fluid durchzulassen, um zu verhindern, dass das von der Seite des Ballons einströmende Fluid zu der Seite der Ballon-Steuervorrichtung strömt.

2. Ballon-Steuervorrichtung (100) nach Anspruch 1, wobei die Gas-/Flüssigkeits-Trenneinrichtung (150, 180, 200) ein Gas-/Flüssigkeits-Trennfilter, ein wasserabsorbierendes Polymerelement, ein Papierfaserelement, ein wasserabsorbierendes Faserelement oder ein Flüssigkeits-Vorratsbehälter ist.

3. Ballon-Steuervorrichtung (100) nach Anspruch 1, wobei die Röhre (64, 80) mittels eines Luer-Lock-Mechanismus mit der Gas-/Flüssigkeits-Trenneinrichtung verbunden ist.

4. Ballon-Steuervorrichtung (100) nach Anspruch 3, die des Weiteren einen hohlen Abschnitt umfasst, der zum Installieren der Gas-/Flüssigkeits-Trenneinrichtung (150) in dem Körper (102) eingerichtet ist.

5. Ballon-Steuervorrichtung nach Anspruch 1, wobei die Gas-/Flüssigkeits-Trenneinrichtung (200) ausgebildet wird, indem ein wasserabsorbierendes Element an die Innenfläche der Röhre (64, 80) geschichtet wird.

6. Ballon-Steuervorrichtung (100) nach Anspruch 1, die des Weiteren einen Flüssigkeitssensor (210, 220) umfasst, der zum Erfassen von Flüssigkeit in der Gas-/Flüssigkeits-Trenneinrichtung (150) eingerichtet ist.

7. Ballon-Steuervorrichtung (100) nach Anspruch 5, die des Weiteren einen Flüssigkeitssensor (210, 220) zum Erfassen von Flüssigkeit in der Gas-/Flüssigkeits-Trenneinrichtung (150) umfasst.

8. Ballon-Steuervorrichtung (100) nach Anspruch 6, wobei der Flüssigkeitssensor (210, 220) eine Komponente aufweist, die ihre Farbe ändert, wenn Flüssigkeit erfasst wird.

9. Ballon-Steuervorrichtung (100) nach Anspruch 7, **dadurch gekennzeichnet, dass** der Flüssigkeitssensor (210, 220) eine Komponente aufweist, die ihre Farbe ändert, wenn Flüssigkeit erfasst wird.

## Revendications

1. Dispositif de commande de ballonnet (100) configuré pour commander une expansion et une contraction d'un ballonnet (42, 72) fixé à un élément d'une unité d'insertion (12) d'uh endoscope (10) et d'un adaptateur d'insertion (70) en délivrant et en aspirant un gaz au et du ballonnet (42, 72), **caractérisé par le fait qu'**il comprend :
- un corps principal (102) du dispositif de commande de ballonnet (100) ;
- un tube (64, 80) reliant le dispositif de commande de ballonnet (100) au ballonnet (42, 72) ;
- un raccord (191, 193) pour relier le tube (64, 80) et le corps principal (102) ; et
- un séparateur de gaz/liquide (150, 180, 200) fixé au raccord et configuré pour faire passer le gaz sans laisser passer le liquide afin que le fluide s'écoulant en entrée depuis le côté du ballonnet soit empêché de s'écouler vers le côté du dispositif de commande de ballonnet.

2. Dispositif de commande de ballonnet (100) selon la revendication 1, dans lequel le séparateur de gaz/liquide (150, 180, 200) est un dispositif parmi un filtre de séparation gaz/liquide, un élément à polymère absorbant l'eau, un élément à fibres de papier, un élément à fibres absorbant l'eau et un conteneur de réserve de liquide.

3. Dispositif de commande de ballonnet (100) selon la revendication 1, dans lequel le tube (64, 80) est lié au séparateur de gaz/liquide (150) avec un mécanisme de verrou "lure lock" ou antiblocage.

4. Dispositif de commande de ballonnet (100) selon la revendication 3, comprenant, en outre, une portion creuse configurée pour installer le séparateur de gaz/liquide (150) à l'intérieur du corps (102).

5. Dispositif de commande de ballonnet (100) selon la revendication 1, dans lequel le séparateur de gaz/liquide (200) est formé en stratifiant un élément absorbant l'eau sur une surface intérieure du tube (64, 80).

6. Dispositif de commande de ballonnet (100) selon la revendication 1, comprenant, en outre, un détecteur de liquide (210, 220) configuré pour détecter un liquide dans le séparateur de gaz/liquide (150).

7. Dispositif de commande de ballonnet (100) selon la revendication 5, comprenant, en outre, un détecteur de liquide (210, 220) pour détecter un liquide dans le séparateur de gaz/liquide (150).

8. Dispositif de commande de ballonnet (100) selon la revendication 6, dans lequel le détecteur de liquide (210, 220) comporte un composant pour changer de couleur lorsqu'un liquide est détecté.

9. Dispositif de commande de ballonnet (100) selon la revendication 7, **caractérisé en ce que** le détecteur de liquide (210, 220) comporte un composant pour changer de couleur lorsqu'un liquide est détecté.
